(19) Europäisches Patentamt
European Patent Office
Office européen des brevets

(11) Veröffentlichungsnummer : **0 039 440**
**B1**

(12) # EUROPÄISCHE PATENTSCHRIFT

(45) Veröffentlichungstag der Patentschrift : **22.06.83**

(51) Int. Cl.³ : **A 61 K 31/425**, C 07 D277/68

(21) Anmeldenummer : **81102999.0**

(22) Anmeldetag : **18.04.81**

(54) **Substituierte Benzothiazol-2(3H)-one enthaltende Arzneimittel.**

(30) Priorität : **02.05.80 DE 3016816**
**10.05.80 DE 3017977**

(43) Veröffentlichungstag der Anmeldung : **11.11.81 Patentblatt 81/45**

(45) Bekanntmachung des Hinweises auf die Patenterteilung : **22.06.83 Patentblatt 83/25**

(84) Benannte Vertragsstaaten :
**BE CH DE FR GB IT LI LU NL SE**

(56) Entgegenhaltungen :
**EP A 0 022 213**
**FR A 2 143 362**
**DIE PHARMAZIE, Band 19, Nr. 4, April 1964, Seiten 281-283 Berlin, DD. W. NIMMICH : « Über die antimikrobielle Wirksamkeit des Benzthiazolons und seiner Derivate »**

(73) Patentinhaber : **Dr. Karl Thomae GmbH**
**Postfach 1755**
**D-7950 Biberach (Riss) (DE)**

(72) Erfinder : **Engel, Wolfhard, Dr. Dipl.-Chem.**
**Mozartstrasse 13**
**D-7950 Biberach 1 (DE)**
Erfinder : **Trummlitz, Günter, Dr. Dipl.-Chem.**
**Buchenweg 27**
**D-7951 Warthausen 1 (DE)**
Erfinder : **Eberlein, Wolfgang, Dr. Dipl.-Chem.**
**Obere Au 6**
**D-7950 Biberach 1 (DE)**
Erfinder : **Schmidt, Günther, Dr. Dipl.-Chem.**
**Johann-Sebastian-Bach-Strasse 27**
**D-7950 Biberach 1 (DE)**
Erfinder : **Engelhardt, Günther, Prof. Dr.**
**Unterer Bühl 18**
**D-7950 Biberach 1 (DE)**
Erfinder : **Zimmermann, Rainer, Dr. Dipl.-Biochem**
**Laurenbühlstrasse 17**
**D-7951 Mittelbiberach (DE)**

**EP 0 039 440 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte Benzothiazol-2 (3H)-one enthaltende Arzneimittel

Die Erfindung betrifft Arzneimittel, die in 4-, 5-, 6- oder 7-Stellung mono- oder in 5,6-Stellung disubstituierte Benzothiazol-2 (3H)-one der allgemeinen Formel 1

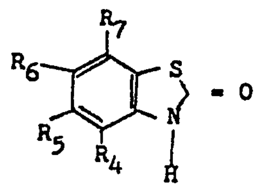

(I)

enthalten. In der allgemeinen Formel I bedeuten :

Entweder $R_5$ die Methoxygruppe, $R_4$, $R_6$ und $R_7$ Wasserstoffatome
oder $R_6$ das Fluoratom, $R_4$, $R_5$ und $R_7$ Wasserstoffatome
oder $R_7$ das Chloratom, $R_4$, $R_5$ und $R_6$ Wasserstoffatome
oder $R_6$ das Chloratom, $R_4$, $R_5$ und $R_7$ Wasserstoffatome
oder $R_5$ und $R_6$ jeweils die Methoxygruppe, $R_4$ und $R_7$ ein Wasserstoffatom
oder $R_4$ die Methylgruppe, $R_5$, $R_6$ und $R_7$ Wasserstoffatome.

Die Verbindungen sind durch folgende Publikationen, die sich mit ausschließlich chemischen oder physikalischen Aspekten der Verbindungen befassen, bekannt :

a) D. Simov und A Antonova, God. Sofii. Univ., Khim. Fak. *67*, 287-293 (1972-1973 ; publiziert 1976) ; C.A. *86*, 154 756p (1977) : Die Arbeit erwähnt u. a. 5-Methoxy-2 (3H)-benzothiazolon und 6-Chlor-2 (3H)-benzothiazolon. Inhaltlich ähnlich ist die Arbeit von A. Antonova und D. Simov, Mh. Chem. *107*, 1487-1491 (1976) ;

b) R.C. Elderfield und F.W. Short, J. Org. Chem. *18*, 1092-1103 (1953). Die Arbeit befaßt sich u. a. mit 7-Chlor-2 (3H)-benzothiazolon ;

c) A.F. Aboulezz, S.M.A. Zayed, W.S. EL-Hamouly und M.L. EL-Sheikh, Egypt. J. Chem. *16*, 355-359 (1973) ; C.A. 81 152 075p (1974), eine Publikation, die ein Verfahren zur Synthese von u. a. 5,6-Dimethoxy-2 (3H)-benzothiazolon angibt ;

d) R.F. Hunter und E.R. Parken, J. Chem. Soc. *1935*, 1755-1761 : sie beschreiben u. a. 6-Chlor-2 (3H)-benzothiazolon.

Darüber hinaus werden 6-Fluor-2(3H)-benzothiazolon und 6-Chlor-2(3H)-benzothiazolon in einer Arbeit von G. Mazzone und G. Pappalardo, Jl Farmaco, Ed. Sc. *32*, 348-354 (1977), beschrieben, die sich mit der Synthese potentieller Antimitotica befaßt.

Es wurde nun überraschenderweise gefunden, daß die Verbindungen der allgemeinen Formel I eine sehr gute analgetische Wirkung neben einer ausgeprägten antipyretischen Wirkung bei einer guten Verträglichkeit aufweisen.

Die Verbindungen der allgemeinen Formel I lassen sich wie folgt herstellen :

1. Ein 2-Amino-thiophenol der allgemeinen Formel II,

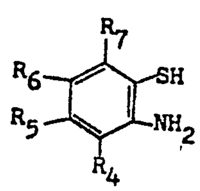

(II)

in der $R_4$ bis $R_7$ wie oben definiert sind, wird mit N,N'-Carbonyldiimidazol in einem wasserfreien Lösungsmittel wie Benzol, Toluol oder anderen Kohlenwasserstoffen oder wie Tetrahydrofuran, Dioxan oder ähnlichen cyclischen Äthern bei Temperaturen bis zum Siedepunkt des Reaktionsgemisches umgesetzt. Im allgemeinen genügt es, zur Aufarbeitung das Lösungsmittel im Vakuum zu entfernen und das Reaktionsprodukt beispielsweise aus 1,2-Dichlorethan, Ethanol oder Methanol umzukristallisieren.

2. Ein in 2-Stellung durch ein Chlor- oder Bromatom oder eine Alkoxygruppe substituiertes Benzothiazol der allgemeinen Formel III,

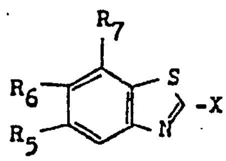

(III)

in der $R_4$ bis $R_7$ wie oben definiert sind und X ein Chlor- oder Bromatom oder eine bis zu 3

Kohlenstoffatomen enthaltende Alkoxygruppe bedeutet, wird bei Temperaturen zwischen 0 und 120 °C, vorzugsweise bei der Siedetemperatur des Reaktionsgemisches, durch Behandlung mit wässerigen Mineralsäuren, beispielsweise mit Konzentrierter Salzsäure oder Bromwasserstoffsäure, 20 bis 60 %iger Schwefelsäure oder halbkonzentrierter Phosphorsäure, zu einer Verbindung der allgemeinen Formel I hydrolysiert. Die Reaktion kann in Gegenwart inerter, mit Wasser mischbarer Lösungsmittel, z. B. Methanol, Ethanol, 1-Propanol, 2-Propanol, Tetrahydrofuran, 1,4-Dioxan, 1,2-Ethandiol durchgeführt werden, sie gelingt jedoch auch bei Abwesenheit eines Lösungsmittels.

3. o-Nitrophenylthioessigsäuren der allgemeinen Formel IV,

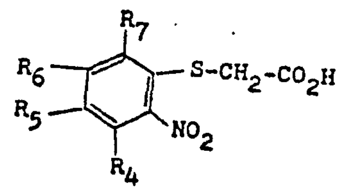

(IV)

in der $R_4$ bis $R_7$ wie eingangs definiert sind, werden durch Erhitzen mit Acetanhydrid- vorzugsweise auf Rückflußtemperatur- zu 3-Acetyl-benzothiazol-2(3H)-onen der allgemeinen Formel V,

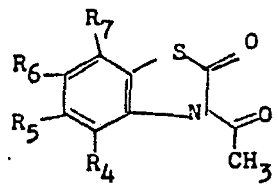

(V)

in der $R_4$ bis $R_7$ wiederum die eingangs genannten Bedeutungen haben, cyclokondensiert. Die Reaktion wird durch Zusatz von Alkaliacetaten, beispielsweise von Natriumacetat, oder von tertiären organischen Basen, beispielsweise von Pyridin oder Triethylamin katalytisch beschleunigt, sie gelingt jedoch mit gleicher Ausbeute auch in Abwesenheit von Katalysatoren.

Die Acetylgruppe in einer Verbindung der allgemeinen Formel V läßt sich durch Umsetzung mit wäßrigen Alkali- oder Erdalkalihydroxiden, z. B. wäßriger Natron-, Kali- oder Barytlauge, mit wäßrigen Aminen oder Ammoniumhydroxid-Lösungen, z. B. konz. Ammoniak oder einer Lösung von Benzyltrimethylammoniumhydroxid, oder durch Behandlung mit wäßrigen Mineralsäuren, beispielsweise konz. Salz- oder Bromwasserstoffsäure, halbkonz. Schwefel- oder Phosphorsäure, abspalten unter Bildung einer entsprechenden Verbindung der allgemeinen Formel I. Für die Hydrolyse eignen sich Temperaturen zwischen 0 und 120 °C, 60 bis 100 °C werden jedoch bevorzugt. Die Reaktion kann mit und ohne mit Wasser mischbaren Cosolventien durchgeführt werden. In Betracht kommende Lösungsmittel sind beispielsweise Alkohole, wie Methanol, Ethanol, 2-Propanol, 1,2-Ethandiol, oder Ether, z. B. 1,4-Dioxan oder Tetrahydrofuran.

Die als Ausgangsmaterialien verwendeten 2-Aminothiophenole der allgemeinen Formel II lassen sich in Analogie zu literaturbekannten Methoden herstellen. So erhält man z. B. 4-Fluor-2-mercapto-anilin (Schmelzpunkt: 139-141 °C) durch Hydrolyse von 6-Fluor-2-benzothiazolamin mit siedender wäßriger Kalilauge (analog R. L. Mital und S. K. Jain, J. chem. Soc. (C) *1969*, 2148 bzw. S. K. Jain and R. L. Mital, Z. Naturforsch. *32B*, 821 [1977] bzw. H. Hauser, Helv. Chim. Acta *11*, 208 [1928] bzw. R. Schuloff u. a., Ber. dtsch. chem. Ges. *61*, 2541 [1928]).

Die Ausgangsmaterialien der allgemeinen Formel III, in der X ein Chlor- oder Bromatom bedeutet, lassen sich aus entsprechenden 2-Benzothiazolaminen durch Umsetzung mit warmer konzentrierter Halogenwasserstoffsäure und Natriumnitrit bereiten. (G. Mazzone und G. Pappalardo, Il Farmaco, Ed. Sc. *32*, 348-354 [1977]). So erhält man beispielsweise aus 6-Chlor-2-benzothiazolamin das 2,6-Dichlorbenzothiazol (Schmelzpunkt: 99-101 °C) und aus 6-Fluor-2-benzothiazolamin das 2-Chlor-6-fluorbenzothiazol (Schmelzpunkt : 92-93 °C).

Die Ausgangsmaterialien der allgemeinen Formel III, in der X eine Alkoxygruppe ist, sind aus entsprechenden N-Aryl-thiourethanen durch Umsetzung mit heißer wäßrig-alkoholischer Lösung von Kaliumhexacyanoferrat-(III) und Natriumhydroxid zugänglich (analog R. F. Hunter und E. R. Parken, J. chem. Soc. *1935*, 1755-1761). So werden z. B. hergestellt : 7-Chlor-2-ethoxybenzothiazol (Öl, ohne völlige Reinigung in die nächste Stufe einsetzbar) neben 5-Chlor-2-ethoxy-benzothiazol, Schmelzpunkt 74-75 °C (Petrolether) aus (3-Chlorphenyl)-thiocarbaminsäure-O-ethylester, Schmelzpunkt : 78-79 °C (Methanol/Wasser) ;

6-Chlor-2-ethoxybenzothiazol, Schmelzpunkt : 60-61 °C (Ethanol) aus (4-Chlorphenyl)-thiocarbaminsäure-O-ethylester, Schmelzpunkt : 103-105 °C (Ethanol) ;

5,6-Dimethoxy-2-ethoxybenzothiazol (82 % der Theorie ; ohne Reinigung in die nächste Stufe einsetzbar) aus (3,4-Dimethoxyphenyl)-thiocarbaminsäure-O-ethylester.

Die erforderlichen N-Aryl-thiourethane erhält man aus N-Aryl-isothiocyanaten mit wasserfreiem Ethanol in Gegenwart von Chinolin (R. F. Hunter und E. R. Parken, J. chem. Soc. *1935*, 1755-1761).

Die als Ausgangsmaterialien erforderlichen o-Nitrophenylthioessigsäuren der allgemeinen Formel IV stellt man beispielsweise aus entsprechenden o-Halogen-nitrobenzolen und Thioglykolsäure entweder in siedender wäßrig-alkoholischer Natriumhydroxid-Lösung (analog A. F. Aboulezz, S M. A. Zayed, W. S. El-Hamouly und M. I. El-Sheikh, Egypt. J. Chem. *16*, 355-359 [1973]) oder vorteilhalft in wasserfreiem Dimethylsulfoxid und in Gegenwart von Natriummethylat her. So erhält man beispielsweise aus 4-Chlor-3-nitroaniol :

[(4-Methoxy-2-nitrophenyl)thio]-essigsäure, Schmelzpunkt : 173-174 °C (Methanol) ;

aus 2,3-Dichlornitrobenzol die [(2-Chlor-6-nitrophenyl)thio]-essigsäure, Schmelzpunkt : 110-112 °C (Methanol) ;

aus 2-Chlor-4,5-dimethoxy-nitrobenzol die

[(4,5-Dimethoxy-2-nitrophenyl)thio ]-essigsäure, Schmelzpunkt : 217 °C (Methanol)

Wie bereits eingangs erwähnt, besitzen die Verbindungen der allgemeinen Formel I eine sehr gute analgetische und antipyretische Wirkung bei geringer akuter Toxizität.

So wurden beispielsweise die Verbindungen

5-Methoxy-2(3H)-benzothiazolon = A
6-Fluor-2(3H)-benzothiazolon = B
7-Chlor-2(3H)-benzothiazolon = C
6-Chlor-benzothiazol-2(3H)-on = D
5,6-Dimethoxy-benzothiazol-2(3H)-on = E
4-Methyl-benzothiazol-2(3H)-on = F

auf eine analgetische Wirkung gegenüber dem Entzündungsschmerz der Ratte, eine analgetische Wirkung gegen den durch Wärme ausgelösten Schmerz der Maus, eine die Körpertemperatur der Ratte senkende Wirkung sowie die akute Toxizität an der Maus geprüft.

Methodik

1. Wirkung gegen den Entzündungsschmerz der Ratte

Die Versuchsanordnung entsprach der von RANDALL-SELITTO (Arch. int. Pharmacodyn. *111*, 409 (1957) angegebenen.

Männliche Chbb : THOM-Ratten mit einem Gewicht zwischen 100 und 130 g erhielten eine subplantare Injektion von 0,1 ml einer 5 %igen Suspension von Hefezellen in 5,55 %iger Glukoselösung in eine Hinterpfote. 1 1/2 Stunden bzw. 2 1/4 Stunden nach der Injektion des Phlogisticums erhielten die Tiere verschiedene Dosen der Prüfsubstanz als Verreibung in 1 %iger Methylzellulose (1 ml/100 g Tier) per Schlundsonde beigebracht. Kontrolltiere erhielten entsprechende Volumina des Vehikels.

90 bzw. 45 min. nach Applikation der Prüfsubstanz (d. h. 3 h nach der subplantaren Injektion der Hefesuspension) wurde bei den Ratten die Schmerzschwelle in g Auflagedruck/Pfote bestimmt.

Aus der nach den verschiedenen Dosen der Prüfsubstanz gemessenen Schmerzschwelle wurde nach linearer Regressionsanalyse nach LINDER (Statistische Methoden, 4. Aufl. pp. 148-162, Birkhäuser, Basel 1964) eine $ED_{50}$ mit den Vertrauensgrenzen nach FIELLER (Quart. J. Pharm. Pharmacol. *17*, 117 (1944)) als jene Dosis ermittelt, die eine Anhebung der Schmerzschwelle um 50 % gegenüber der der Kontrollen bewirkte.

2. Wirkung gegen den Wärmeschmerz der Maus

Die Prüfung erfolgte mit Hilfe einer Modifikation der Versuchsanordnung nach CHEN u. BECKMAN (Science *113*, 631 (1951) an männlichen Chbb : NMRI (SPF)-Mäusen mit einem mittleren Gewicht von 20 g. Die « heiße Platte » bestand aus Aluminium und hatte eine Oberflächentemperatur von 52 °C.

Die Prüfsubstanzen wurden als Verreibung in 1 %iger Methylzellulose (0,1 ml/10 g Maus) per Schlundsonde beigebracht. Vor der Behandlung mit Prüfsubstanz wurden die Tiere im Abstand von 30 min. zweimal auf die heiße Platte gesetzt. Es wurde ihre individuelle Reaktionszeit ermittelt. Nach Gabe der Prüfsubstanz wurde in Abständen von 30 min. die Reaktionszeit erneut gemessen.

Aus der mit den verschiedenen Dosen der Prüfsubstanzen erzielten gemittelten maximalen Verlängerung der Reaktionszeit wurde nach linearer Regressionsanalyse nach LINDER (s. o.) eine $ED_{100}$ mit den Vertrauensgrenzen nach FIELLER (s. o.) als die Dosis berechnet, die zu einer 100 %igen Verlängerung der Reaktionszeit führt.

3. Wirkung auf die Körpertemperatur der Ratte

Die Prüfung der temperatursenkenden Wirkung erfolgte durch die Kontrolle des Verlaufes der rektal gemessenen Körpertemperatur von Chbb : THOM-Ratten mit einem Gewicht zwischen 125-150 g über im

Rektum verweilende Thermoelemente. Die Prüfsubstanzen wurden als Verreibung in 1 %iger Methylzellulose (1,0 ml/100 g Tier) per Schlundsonde beigebracht. Aus den nach Gabe der verschiedenen Dosen der Prüfsubstanz gewonnenen Werten für die mittlere maximale Temperatursenkung wurde nach linearer Regressionsanalyse nach LINDER (s. o.) eine $ED_{-1,5\,°C}$ als jene Dosis berechnet, die eine Senkung der Körpertemperatur um 1,5 °C bewirkte.

4. Akute Toxizität an der Maus

Die Bestimmung der akuten Toxizität erfolgte an Chbb : NMRI (SPF)-Mäusen beider Geschlechter mit einem mittleren Gewicht von 20 g. Die Prüfsubstanzen wurden als Verreibung in 1 %iger Methylzellulose (0,5 ml/10 g Tier) per Schlundsonde verabfolgt. Die Berechnung der $LD_{50}$ erfolgte nach LITCHFIELD u. WILCOXON (J. Pharmacol. exp. Therap. *96,* 99 (1949)) aus dem Prozentsatz der Tiere, die nach den verschiedenen Dosen innerhalb von 14 Tagen verstarben.

Ergebnisse

Die bei diesen Prüfungen erzielten Befunde sind in den Tabellen 1 bis 5 zusammengefaßt.

Tabelle 1

Wirkung gegen den Entzündungsschmerz der Ratte in der Versuchsanordnung nach RANDALL u. SELITTO 45 min nach oraler Gabe

| Substanz | $ED_{50}$ mg/kg | Vertrauensgrenzen bei 95 % Wahrscheinlichkeit |
|---|---|---|
| A | 90 | 84-95 |
| B | 121 | 114-129 |
| C | 106 | 99-113 |
| D | 175 | 153-208 |
| E | 43 | 40-45 |
| F | 174 | 162-186 |

Tabelle 2

Wirkung gegen den Entzündungsschmerz der Ratte in der Versuchsanordnung nach RANDALL u. SELITTO 90 min nach oraler Gabe

| Substanz | $ED_{50}$ mg/kg | Vertrauensgrenzen bei 95 % Wahrscheinlichkeit |
|---|---|---|
| A | 150 | 142-157 |
| B | 76 | 71-80 |
| C | 105 | 92-120 |
| D | 129 | 122-138 |
| E | 40 | 38-42 |
| F | 202 | 187-217 |

Tabelle 3

Wirkung gegen den Wärmeschmerz der Maus auf der « heißen Platte » nach oraler Gabe

| Substanz | $ED_{100}$ mg/kg | Vertrauensgrenzen bei 95 % Wahrscheinlichkeit |
|---|---|---|
| A | 218 | 127-259 |
| B | 165 | 140-261 |
| E | 92 | 62-127 |
| F | 113 | 100-124 |

Tabelle 4

Wirkung auf die Körpertemperatur der normothermen Ratte nach oraler Gabe

| Substanz | $ED_{-1,5\,°C}$ mg/kg | Vertrauensgrenzen bei 95 % Wahrscheinlichkeit |
|---|---|---|
| A | 58 | 48-77 |
| B | 83 | 71-100 |
| D | 103 | 80-142 |
| E | 29 | 25-33 |
| F | 118 | 80-157 |

Tabelle 5

Akute Toxizität an der Maus nach oraler Gabe bei einer Nachbeobachtungszeit von 14 Tagen

| Substanz | $LD_{50}$ mg/kg | Vertrauensgrenzen bei 95 % Wahrscheinlichkeit |
|---|---|---|
| A | 1 600 | 1 310-1 950 |
| B | 1 650 | 1 320-2 062 |
| C | 10 000 [1)] | |
| F | 8 000 [2)] | |

1) Diese Dosis wurde von allen 10 Tieren überlebt.
2) Nach dieser Dosis starben 4 von 20 Tieren.

Die geprüften Verbindungen zeichnen sich durchweg bei einer sehr geringen Toxizität durch gute analgetische und antipyretische Wirkungsqualitäten aus.

Die nachfolgenden Beispiele sollen die Erfindung näher beschreiben :

Beispiel 1

6-Fluor-2(3H)-benzothiazolon

Zu der Lösung von 194,5 g (1,75 mol) 4-Fluor-2-mercapto-anilin in 2,5 l wasserfreiem Tetrahydrofuran gibt man unter Rühren und unter Einhaltung einer Temperatur zwischen 45 und 50 °C portionsweise 300,0 g (1,87 mol) N,N'-Carbonyldiimidazol und kocht anschließend 30 Minuten unter Rückfluß. Vom Lösungsmittel werden 1,7 l abdestilliert, die verbleibende Lösung wird heiß filtriert und in 3 l Wasser eingerührt. Nach 1/2 Stunde wird das ausgefallene Produkt abgenutscht und mit Wasser gründlich gewaschen. Man kristallisiert aus Ethanol und 1,2-Dichlorethan, jeweils unter Verwendung von Aktivkohle, um und erhält 95,3 g (32 % der Theorie) an farblosen Kristallen vom Schmelzpunkt 188-189 °C.

$C_7H_4FNOS$ (169,17)

| | C | H | N | S |
|---|---|---|---|---|
| Ber. : | 49,70 | 2,38 | 8,28 | 18,95 |
| Gef. : | 49,55 | 2,46 | 8,45 | 19,00 |

Beispiel 2

5-Methoxy-2(3H)-benzothiazolon

a) 3-Acetyl-5-methoxy-2(3H)-benzothiazolon

Die Lösung von 24,5 g (0,100 7 mol) [(4-Methoxy-2-nitrophenyl)thio]-essigsäure in 150 ml Acetanhydrid wird 6 Stunden unter Rückfluß gekocht. Das überschüssige Acetanhydrid wird im Wasserstrahlvakuum abdestilliert, der verbleibende Rückstand zwischen Wasser und Dichlormethan verteilt. Die Methylenchloridphase wird eingedampft, der Rückstand an Kieselgel unter Verwendung von Dichlormethan/Essigsäureethylester (Volumenverhältnis 1 : 1) zum Eluieren chromatographisch gereinigt, schließlich aus Diisopropylether/Essigsäureethylester (1 : 1) unter Verwendung von Aktivkohle umkristallisiert. Man erhält 10,4 g (46 % der Theorie) an farblosen Kristallen vom Schmelzpunkt 112-113 °C.

$C_{10}H_9NO_3S$ (223,25)
Ber.: C 53,80 H 4,06 N 6,27 S 14,36
Gef.: 53,84 4,26 6,31 14,60

b) 5-Methoxy-2(3H)-benzothiazolon

7,0 g (0,0314 mol) 3-Acetyl-5-methoxy-2(3H)-benzothiazolon werden in einer Mischung aus 210 ml 5 N Salzsäure und 70 ml Ethanol suspendiert und 1 Stunde unter Rückfluß gekocht. Die noch heiße Mischung wird filtriert, mit 200 ml Wasser verdünnt und durch äußere Kühlung mit Eis auf eine Temperatur von + 5 °C gebracht. Nach 2 stündigem Stehenlassen wird der Niederschlag abfiltriert, das getrocknete Produkt an Kieselgel unter Verwendung von 1,2-Dichlorethan/Aceton (Volumenverhältnis 9 : 1) zum Eluieren chromatographisch gereinigt, zuletzt aus Methanol und aus 1,2-Dichlorethan, jeweils unter Zusatz von Aktivkohle, umkristallisiert. Die in einer Ausbeute von 3,25 g (57 % der Theorie) erhaltenen schwach gelben Kristalle schmelzen bei 168-170 °C.
$C_8H_7NO_2S$ (181,21)
Ber.: C 53,03 H 3,89 N 7,73 S 17,69
Gef.: 53,20 3,90 7,74 17,70

Beispiel 3

7-Chlor-2(3H)-benzothiazolon

a) 3-Acetyl-7-chlor-2(3H)benzothiazolon

Die Mischung aus 79,0 g (0,32 mol) [(2-Chlor-6-nitrophenyl)thio]-essigsäure, 150,0 g (1,83 mol) wasserfreiem Natriumacetat und 600 ml Acetanhydrid wird 30 Minuten auf 100 °C erhitzt. Man destilliert das Acetanhydrid im Wasserstrahlvakuum ab, verrührt den Rückstand mit 1 l Wasser und nutscht das angefallene Produkt ab. Man reinigt durch Chromatographieren an Kieselgel unter Verwendung von Dichlormethan/Essigsäureethylester (Volumenverhältnis 1 : 1) zum Eluieren, kristallisiert abschließend aus Ethanol um und erhält 19,2 g (26 % der Theorie) an farblosen kristallen vom Schmelzpunkt 74 °C.

b) 7-Chlor-2(3H)-benzothiazolon

Hergestellt analog Beispiel 2b) aus 3-Acetyl-7-chlor-2(3H)-benzothiazolon in einer Ausbeute von 90 % der Theorie. Schmelzpunkt: 202-203 °C (1,2-Dichlorethan)
$C_7H_4ClNOS$ (185,63)
Ber.: C 45,29 H 2,17 Cl 19,10 N 7,55 S 17,27
Gef.: 45,24 2,26 19,40 7,64 17,20

Beispiel 4

5,6-Dimethoxy-2(3H)-benzothiazolon

a) 3-Acetyl-5,6-dimethoxy-2(3H)-benzothiazolon

Hergestellt analog Beispiel 2a) aus [(4,5-Dimethoxy-2-nitrophenyl)thio]-essigsäure und Acetanhydrid in einer Ausbeute von 34 % der Theorie.
Schmelzpunkt: 164-165 °C (Diisopropylether/Essigsäureethylester).

b) 5,6-Dimethoxy-2(3H)-benzothiazolon

2,533 g (0,01 Mol) 3-Acetyl-5,6-dimethoxy-2(3H)-benzothiazolon werden in der Mischung aus 40 ml Ethanol und 50 ml konzentriertem Ammoniak 2 Stunden unter Rückfluß gekocht. Man dampft die Mischung auf ein Viertel ihres ursprünglichen Volumens ein, läßt erkalten, filtriert und reinigt den getrockneten Niederschlag durch Chromatographieren an Kieselgel unter Verwendung von 1,2-Dichlorethan/Aceton (Volumenverhältnis 9 : 1) zum Eluieren. Die eingedampften Eluate werden aus Methanol unter Verwendung von Aktivkohle umkristallisiert und ergeben 1,78 g (84 % der Theorie) des gewünschten Produktes vom Schmelzpunkt 192-193 °C.
$C_9H_9NO_3S$ (211,25)
Ber.: C 51,17 H 4,29 N 6,63 S 15,18
Gef.: 51,27 4,42 6,86 15,22

Beispiel 5

5,6-Dimethoxy-2(3H)-benzothiazolon

19,7 g (82 mmol) 5,6-Dimethoxy-2-ethoxybenzothiazol werden in 500 ml Ethanol gelöst und in der Siedehitze tropfenweise mit 64 ml konzentrierter wäßriger Salzsäure versetzt. Man kocht 3 Stunden unter Rückfluß and dampft anschließend das Lösungsmittel ab. Der Rückstand wird aus Methanol umkristallisiert. Man erhält 8,1 g (48 % der Theorie) an 5,6-Dimethoxy-2(3H)-benzothiazolon vom Schmelzpunkt 191 °C.

$C_9H_9NO_3S$ (211,25)

| | | | | |
|---|---|---|---|---|
| Ber. : | C 51,17 | H 4,29 | N 6,63 | S 15,18 |
| Gef. : | 51,20 | 4,24 | 6,53 | 15,40 |

## Beispiel 6

6-Chlor-2(3H)-benzothiazolon

Hergestellt analog Beispiel 5 aus 6-Chlor-2-ethoxy-benzothiazol und konzentrierter Salzsäure in einer Ausbeute von 56 % der Theorie.

Schmelzpunkt : 212-213 °C (Toluol)

$C_7H_4ClNOS$ (185,63)

| | | | | | |
|---|---|---|---|---|---|
| Ber. : | C 45,29 | H 2,17 | Cl 19,10 | N 7,55 | S 17,27 |
| Gef. : | 45,40 | 2,23 | 19,00 | 7,63 | 17,36 |

## Beispiel 7

7-Chlor-2(3H)-benzothiazolon

a) (3-Chlorphenyl)-thiocarbaminsäure-O-ethylester

Die Mischung aus 45,5 g (0,268 mol) 3-Chlorphenylisothiocyanat, 200 ml wasserfreiem Ethanol und 1 ml Chinolin wird 48 Stunden unter Rückfluß gekocht. Danach wird das Reaktionsgemisch im Wasserstrahlvakuum eingedampft ; der Rückstand wird in Ether aufgenommen ; die resultierende Lösung mit 5 %iger Salzsäure, danach mit Wasser gründlich gewaschen, getrocknet und abermals eingedampft. Nach dem Umkristallisieren aus Methanol/Wasser erhält man 49,5 g (86 % der Theorie) an farblosen Kristallen vom Schmelzpunkt 78-79 °C.

b) 7-Chlor-2-ethoxybenzothiazol

In eine auf 80 bis 90 °C erwärmte Lösung von 494 g (1,5 mol) Kaliumhexacyanoferrat (III) in 600 ml Wasser wird eine Mischung aus 49,5 g (0,23 mol) (3-Chlorphenyl)-thiocarbaminsäure-O-ethylester, 60 ml Ethanol, 350 ml 30 proz. wäßriger Natronlauge und 300 ml Wasser in 20 ml-Portionen in Abständen von je 5 Minuten zugegeben. Anschließend erhitzt man 2 Stunden auf 90 °C, gibt nochmals 200 g (0,61 mol) Kaliumhexacyanoferrat (III) zu und erwärmt weitere 3 Stunden auf 90 °C. Nach dem Erkalten verdünnt man mit 3 l Wasser, extrahiert die Mischung erschöpfend mit Ether, trennt, wäscht die organische Schicht mit Wasser und trocknet sie über Natriumsulfat. Der nach dem Abdampfen des Lösungsmittels verbleibende Rückstand wird aus 500 g Kieselgel unter Verwendung von Dichlormethan/Petrolether (Volumenverhältnis 1 : 1) zum Eluieren chromatographisch gereinigt. Die die beiden Hauptprodukte enthaltenden Fraktionen werden vereinigt und eingedampft. Der zurückbleibende Kristallbrei ergibt nach zweimaligem Umkristallisieren aus Petrolether 7,5 g an farblosen Kristallen vom Schmelzpunkt 74-75 °C, die nach spektroskopischen Untersuchungen aus 5-Chlor-2-ethoxybenzothiazol bestehen.

Zur Isolierung des gesuchten 7-Chlor-2-ethoxybenzothiazols, der Substanz mit dem kleineren $R_f$-Wert, werden die vereinigten Mutterlaugen nochmals an 500 g Kieselgel unter Verwendung von Petrolether/Dichlormethan chromatographiert. Das nach dem Eindampfen der entsprechenden Eluate erhaltene 7-Chlor-2-ethoxybenzothiazol, ein schwach gelbes Öl, wird ohne weitere Reinigung in der nächsten Stufe verwendet. Ausbeute : 15,5 g (32 % der Theorie).

c) 7-Chlor-2(3H)-benzothiazolon

Hergestellt analog Beispiel 5 aus 7-Chlor-2-ethoxybenzothiazol und konzentrierter Salzsäure in einer Ausbeute von 38 % der Theorie.

Schmelzpunkt : 202-203 °C (nach dem Umkristallisieren aus Methanol und aus Diisopropylether).

$C_7H_4ClNOS$ (185,63)

| | | | | |
|---|---|---|---|---|
| Ber. : | C 45,29 | H 2,17 | Cl 19,10 | S 17,27 |
| Gef. : | 45,15 | 2,19 | 19,23 | 17,43 |

## Beispiel 8

6-Chlor-2(3H)-benzothiazolon

4,081g (0,02 mol) 2,6-Dichlorbenzothiazol werden in 100 ml einer Mischung aus gleichen Teilen Ethanol und konzentrierter Salzsäure 4 Stunden unter Rückfluß gekocht. Man verdünnt mit 100 ml Wasser, filtriert den erhaltenen Niederschlag ab und wäscht ihn gründlich mit Wasser. Zur weiteren Reinigung nimmt man in 10 proz. Natronlauge auf, filtriert und ethert die Filtrate zweimal mit je 100 ml Ether aus. Die wäßrige Phase wird mit Salzsäure angesäuert, der anfallende Niederschlag abgenutscht, mit Wasser gewaschen und einmal aus Ethanol, einmal aus Toluol, jeweils unter Verwendung von Aktivkohle, umkristallisiert. Man erhält 3,1 g (83 % der Theorie) an Kristallen vom Schmelzpunkt 212-213 °C, nach Mischschmelzpunkt, Elementaranalyse und dünnschichtchromatographischer Untersuchung identisch mit einem nach Beispiel 6 erhaltenen Produkt.

Beispiel 9

6-Fluor-2(3H)-benzothiazolon

Hergestellt analog Beispiel 8 aus 2-Chlor-6-fluorbenzothiazol und konzentrierter Salzsäure in einer Ausbeute von 45 % der Theorie.

Schmelzpunkt : 189-190 °C (1,2-Dichlorethan).

Das Produkt ist nach Mischschmelzpunkt, Elementaranalyse und IR-Spektrum identisch mit einem nach Beispiel 1 erhaltenen Produkt.

Beispiel 10

4-Methyl-2(3H)-benzothiazolon

Hergestellt analog Beispiel 5 aus 2-Ethoxy-4-methylbenzothiazol und konzentrierter Salzsäure in einer Ausbeute von 81 % der Theorie.

Fp. 207-208 °C (Xylol/Benzin 1 : 1) ;

$C_8H_7NOS$ (165.21)

| | | | | |
|---|---|---|---|---|
| Ber. : | C 58,16 | H 4,27 | N 8,48 | S 19,41 |
| Gef. : | 57,72 | 4,24 | 8,50 | 19,45 |

Die Verbindungen der allgemeinen Formel I lassen sich in die üblichen pharmazeutischen Zubereitungsformen, wie Tabletten, Dragées, Suppositorien, Kapseln oder Säfte einarbeiten. Die Einzeldosierung beträgt hierbei 20 bis 600 mg, für Erwachsene vorzugsweise 50 bis 300 mg, dies entspricht einer Tagesdosierung von 60 bis 1 800 mg, vorzugsweise von 180 bis 900 mg.

Die nachfolgenden Beispiele sollen die Herstellung einiger pharmazeutischer Zubereitungsformen verdeutlichen.

Beispiel I

Tabletten mit 50 mg 5-Methoxy-benzothiazol-2(3H)-on

Zusammensetzung :

1 Tablette enthält :

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

Herstellungsverfahren :

Aus Kartoffelstärke wird durch Erwärmen ein 10 %iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

Tablettengewicht : 220 mg

Stempel : 9 mm

Beispiel II

Dragées mit 50 mg 5-Methoxy-benzothiazol-2(3H)-on

Die nach Beispiel I hergestellten Tabletten werden nach bekannten Verfahren mit einer Hülle überzogen, die im wesentlichen aus Zucker und Talkum besteht. Die fertigen Dragées werden mit Hilfe

von Bienenwachs poliert.
Dragéegewicht : 300 mg

Beispiel III

Kapseln mit 60 mg 5-Methoxy-benzothiazol-2(3H)-on

Zusammensetzung :
1 Kapsel enthält :

| | |
|---|---|
| Wirkstoff | 60,0 mg |

Herstellungsverfahren :

Der Wirkstoff wird mikronisiert und in Kapseln abgefüllt, letztere werden anschließend verschlossen.

Beispiel IV

Suppositorien mit 60 mg 5-Methoxy-benzothiazol-2(3H)-on

Zusammensetzung :
1 Zäpfchen enthält :

| | |
|---|---|
| Wirkstoff | 60,0 mg |
| Zäpfchenmasse (z. B. Witepsol W 45 R) | 1 640,0 mg |
| | 1 700,0 mg |

Herstellungsverfahren :

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht : 1,7 g

Beispiel V

Tabletten zu 200 mg 6-Fluor-benzothiazol-2(3H)-on

1 Tablette enthält :

| | |
|---|---|
| Wirksubstanz | 200,0 mg |
| Milchzucker | 120,0 mg |
| Maisstärke | 70,0 mg |
| Polyvinylpyrrolidon | 8,0 mg |
| Magnesiumsterarat | 2,0 mg |
| | 400,0 mg |

Herstellung :

Die Wirksubstanz, der Milchzucker und die Maisstärke werden mit einer wäßrigen Lösung von Polyvinylpyrrolidon gleichmäßig befeuchtet, durch ein Sieb mit 2 mm-Maschenweite gesiebt und im Umlufttrockenschrank bei 50 °C getrocknet. Nach erneuter Siebung durch 1,5 mm-Maschenweite wird Magnesiumstearat zugemischt und die Mischung zu Tabletten verpreßt.
Tablettengewicht : 400 mg
Durchmesser : 11 mm, rund, biplan, beidseitige Facette und einseitige Teilkerbe.

Beispiel VI

Suppositorien, zu 200 mg 6-Fluor-benzothiazol-2(3)-on

1 Zäpfchen enthält :

| | |
|---|---|
| Wirksubstanz | 0,20 g |
| Hartfett (z. B. Witepsol H 19 oder Witepsol W 45) | 1,50 g |
| | 1,70 g |

Herstellung :

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen

ausgegossen.
Zäpfchengewicht : 1,7 g.

Beispiel VII

Suspension mit 200 mg 5-Methoxy-benzothiazol-2(3)-on

100 ml Suspension enthalten :

| | |
|---|---|
| Wirksubstanz | 4,0 g |
| Carboxymethylcellulose | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70 % | 20,0 g |
| Aroma | 0,3 g |
| Wasser dest. ad | 100,0 ml |

Herstellungsverfahren :

Dest. Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren die Wirksubstanz zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 200 mg Wirksubstanz.

**Ansprüche**

1. Arzneimittel enthaltend ein oder mehrere, in 4-, 5-, 6- oder 7-Stellung mono- oder in 5,6-Stellung disubstituierte Benzothiazol-2(3H)-one der allgemeinen Formel

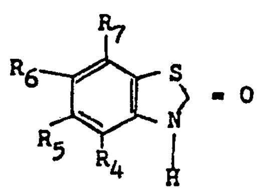

(I)

in der
entweder $R_5$ die Methoxygruppe, $R_4$, $R_6$ und $R_7$ Wasserstoffatome, oder
$R_6$ ein Fluoratom, $R_4$, $R_5$ und $R_7$ Wasserstoffatome oder
$R_7$ ein Chloratom, $R_4$, $R_5$ und $R_6$ Wasserstoffatome oder
$R_6$ ein Chloratom, $R_4$, $R_5$ und $R_7$ Wasserstoffatome, oder
$R_5$ und $R_6$ jeweils die Methoxygruppe, $R_4$ und $R_7$ ein Wasserstoffatom oder
$R_4$ die Methylgruppe, $R_5$, $R_6$ und $R_7$ Wasserstoffatome bedeuten, neben üblichen Träger- und/oder Hilfsstoffen.

2. Arzneimittel enthaltend ein oder mehrere Benzothiazol-2(3H)-one der allgemeinen Formel I gemäß Anspruch 1, neben den üblichen Träger- und/oder Hilfsstoffen, dadurch gekennzeichnet, daß in der allgemeinen Formel I entweder
$R_6$ ein Chloratom, $R_4$, $R_5$ und $R_7$ Wasserstoffatome, oder
$R_7$ ein Chloratom, $R_4$, $R_5$ und $R_6$ Wasserstoffatome oder
$R_5$ eine Methoxygruppe, $R_4$, $R_6$ und $R_7$ Wasserstoffatome bedeuten.

3. Arzneimittel enthaltend 6-Chlor-2(3H)-benzothiazolon neben üblichen Träger- und/oder Hilfsstoffen.

4. Arzneimittel enthaltend 7-Chlor-2(3H)-benzothiazolon neben üblichen Träger- und/oder Hilfsstoffen.

5. Arzneimittel enthaltend 5-Methoxy-2(3H)-benzothiazolon neben üblichen Träger- und/oder Hilfsstoffen.

6. Arzneimittel zur Bekämpfung von Schmerzzuständen, enthaltend 6-Chlor-2(3H)-benzothiazolon und/oder 7-Chlor-2(3H)-benzothiazolon neben üblichen Träger- und/oder Hilfsstoffen.

7. Arzneimittel enthaltend eine Verbindung der allgemeinen Formel I gemäß Anspruch 1, 2 oder 6 als einzigen Wirkstoff neben üblichen Träger- und/oder Hilfsstoffen.

8. Verbindungen der allgemeinen Formel I gemäß Anspruch 1 zur Anwendung bei der Herstellung

von Arzneimitteln zur Bekämpfung von Schmerzzuständen.

9. Analgetika enthaltend eine oder mehrere Verbindungen der allgemeinen Formel I gemäß Anspruch 1 den üblichen Träger- und/oder Hilfsstoffen.

**Claims**

1. Pharmaceutical compositions containing one or more 4-, 5-, 6- or 7-monosubstituted or 5,6-disubstituted benzothiazol-2(3H)-ones of general formula I

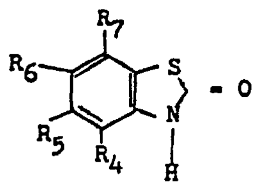 (I)

wherein

either $R_5$ represents a methoxy group and $R_4$, $R_6$ and $R_7$ represent hydrogen atoms ; or
$R_6$ represents a fluorine atom and $R_4$, $R_5$ and $R_7$ represent hydrogen atoms ; or
$R_7$ represents a chlorine atom and $R_4$, $R_5$ and $R_6$ represent hydrogen atoms ; or
$R_6$ represents a chlorine atom and $R_4$, $R_5$ and $R_7$ represent hydrogen atoms ; or
$R_5$ and $R_6$ each represent a methoxy group and $R_4$ and $R_7$ each represent a hydrogen atom ; or
$R_4$ represents a methyl group and $R_5$, $R_6$ and $R_7$ represent hydrogen atoms) together with conventional carriers or excipients.

2. Pharmaceutical compositions containing one or more benzothiazol-2(3H)-ones of general formula I as claimed in claim 1, together with the conventional carriers and/or excipients, characterised in that, in general formula I, either $R_6$ represents a chlorine atom and $R_4$, $R_5$ and $R_7$ represent hydrogen atoms ; or
$R_7$ represents a chlorine atom and $R_4$, $R_5$ and $R_6$ represent a hydrogen atoms ; or
$R_5$ represents a methoxy group and $R_4$, $R_6$ and $R_7$ represent hydrogen atoms.

3. Pharmaceutical compositions containing 6-chloro-2(3H)-benzothiazolone together with conventional carriers and/or excipients.

4. Pharmaceutical compositions containing 7-chloro-2(3H)-benzothiazolone together with conventional carriers and/or excipients.

5. Pharmaceutical compositions containing 5-methoxy-2(3H)-benzothiazolone together with conventional carriers and/or excipients.

6. Pharmaceutical compositions for treating pain, containing 6-chloro-2(3H)-benzothiazolone and/or 7-chloro-2(3H)-benzothiazolone together with conventional carriers and/or excipients.

7. Pharmaceutical compositions containing a compound of general formula I as claimed in claim 1, 2 or 6 as the sole active ingredient, together with conventional carriers and/or excipients.

8. Compounds of general formula I as claimed in claim 1 for use in the preparation of pharmaceutical compositions for the treatment of pain.

9. Analgesics containing one or more compounds of general formula I as claimed in claim 1 together with the conventional carriers and/or excipients.

**Revendications**

1. Médicament contenant une ou plusieurs benzothiazol-2(3H)-ones monosubstituées en position 4, 5, 6 ou 7 ou disubstituées en position 5, 6, de formule générale

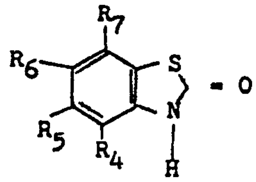 (I)

dans laquelle

ou bien $R_5$ représente le groupe méthoxy, $R_4$, $R_6$ et $R_7$ représentent des atomes d'hydrogène, ou
$R_6$ représente un atome de fluor, $R_4$, $R_5$ et $R_7$ représentent des atomes d'hydrogène, ou
$R_7$ représente un atome de chlore, $R_4$, $R_5$ et $R_6$ représentent des atomes d'hydrogène, ou
$R_6$ représente un atome de chlore, $R_4$, $R_5$ et $R_7$ représentent des atomes d'hydrogène, ou
$R_5$ et $R_6$ représentent chacun le groupe méthoxy, $R_4$ et $R_7$ représentent un atome d'hydrogène ou

$R_4$ représente le groupe méthyle, $R_5$, $R_6$ et $R_7$ représentent des atomes d'hydrogène, conjointement à des excipients et/ou adjuvants habituels.

2. Médicament contenant une ou plusieurs benzothiazol-2 (3H)-ones de formule générale I selon la revendication 1, conjointement aux excipients et/ou adjuvants habituels, caractérisé en ce que dans la formule générale I ou bien $R_6$ représente un atome de chlore, $R_4$, $R_5$ et $R_7$ représentent des atomes d'hydrogène, ou

$R_7$ représente un atome de chlore, $R_4$, $R_5$ et $R_6$ représentent des atomes d'hydrogène, ou

$R_5$ représente un groupe méthoxy, $R_4$, $R_6$ et $R_7$ représentent des atomes d'hydrogène.

3. Médicament contenant de la 6-chloro-2 (3H)-benzothiazolone conjointement à des excipients et/ou adjuvants habituels.

4. Médicament contenant de la 7-chloro-2 (3H)-benzo-thiazolone conjointement à des excipients et/ou adjuvants habituels.

5. Médicament contenant de la 5-méthoxy-2 (3H)-benzothiazolone conjointement à des excipients et/ou adjuvants habituels.

6. Médicament pour combattre les états douloureux, contenant de la 6-chloro-2 (3H)-benzothiazolone et/ou de la 7-chloro-2 (3H)-benzothiazolone conjointement à des excipients et/ou adjuvants habituels.

7. Médicament contenant un composé de formule générale I selon la revendication 1, 2 ou 6 en tant que substance active unique conjointement à des excipients et/ou adjuvants habituels.

8. Composés de formule générale I selon la revendication 1 pour l'utilisation dans la fabrication de médicaments pour combattre les états douloureux.

9. Analgésique contenant un ou plusieurs composés de formule générale I selon la revendication 1 conjointement aux excipients et/ou adjuvants habituels.